# Europäisches Patentamt
# European Patent Office
# Office européen des brevets

(19)

(11) Numéro de publication: **0 247 371**
**A1**

(12) ## DEMANDE DE BREVET EUROPEEN

(21) Numéro de dépôt: **87106172.7**

(22) Date de dépôt: **28.04.87**

(51) Int. Cl.⁴: **A 61 M 25/00, A 61 B 1/00**

(30) Priorité: 23.05.86 CH 2083/86
28.01.87 CH 289/87

(43) Date de publication de la demande: **02.12.87**
**Bulletin 87/49**

(84) Etats contractants désignés: **AT BE CH DE ES FR GB GR IT LI LU NL SE**

(71) Demandeur: **SARCEM SA, 27 rue Cardinal-Journet Case Postale 371, CH-1217 Meyrin 1 (CH)**

(72) Inventeur: **Bonello, Philippe, 26, Chemin du Grand-Pommier, CH-1218 Grand-Saconnex (CH)**
Inventeur: **Jeanmonod, Maurice, 47, Avenue de Vaudagne, CH-1217 Meyrin (CH)**

(74) Mandataire: **Micheli, Michel-Pierre, MICHELI & CIE 118, Rue du Rhône Case Postale 47, CH-1211 Genève 6 (CH)**

(54) **Cathéter-guide.**

(57) Il comprend une tête (3) formée par un ressort à boudin (4) présentant une zone médiane (6) où les pires de ce ressort sont disjointes, l'extrémité libre de cette tête (3) étant obturée. Un élément tubulaire (2) relie cette tête à un boîtier de commande (1) auquel il est fixé de façon amovible. Un dispositif de commande (14, 15) de la flexion de la tête (3) comporte un organe de manœuvre (14) coulissant dans le boîtier (1) relié par un organe de traction (15) à un point excentré de l'extrémité libre de la tête (3). Un dispositif de sectionnement (17 à 21) de l'organe de traction (15) est logé dans le boîtier (1). L'élément tubulaire (2) est constitué par un ressort à boudin (4) à spires jointives recouvert d'un revêtement étanche (9) en matière plastique.

## Cathéter-guide

La présente invention a pour objet un cathéter-guide à commande à distance principalement destiné aux soins des maladies cardio-vasculaires.

On connaît de la demande de brevet EP-86115566.1 (non encore publiée) un cathéter-guide comprenant une tête formée d'un ressort spiral à spires jointives à ses deux extrémités et à spires disjointes dans sa partie médiane dont une extrémité est obturée par une bouterolle tandis que l'autre est fixée sur un embout, lui-même fixé sur l'extrémité d'un tube fin et flexible. L'autre extrémité de ce tube fin et flexible est reliée de façon amovible à un boîtier de commande. Un fil de traction fixé de façon excentrée à la bouterolle de la tête traverse celle-ci ainsi que le tube fin et flexible et est fixé à un organe de traction du boîtier de commande. Ainsi par des tractions sur ce fil plus ou moins fortes, on peut donner à la tête du cathéter-guide une courbure plus ou moins accentuée. Le boîtier de commande comporte encore des moyens permettant de sectionner le fil de traction une fois le cathéter-guide mis en place et avant de séparer ce boîtier de commande du tube fin et flexible. Enfin ce boîtier de commande comporte encore des moyens permettant d'introduire dans le tube fin et flexible un liquide de contraste qui peut s'échapper dans un vaisseau sanguin entre les spires disjointes du ressort à boudin de la tête du cathéter-guide.

Un tel cathéter-guide est généralement de très faible diamètre, plus faible que celui d'un cathéter classique et peut ainsi être plus facilement enfilé au-travers du réseau sanguin d'un patient jusqu'à ce que sa tête atteigne une sténose à soigner. Lorsque cette opération est réalisée, le boîtier de commande est séparé du tube fin et flexible et une rallonge pleine est emboîtée sur l'extrémité libre de ce tube. Ce cathéter guide constitue dès cet instant un moyen de guidage ou rail pour cathéter tubulaire qui est enfilé sur le cathéter-guide et qui peut ainsi, malgré son plus grand diamètre, être facilement mis en place pour le traitement proprement dit de la sténose.

De tels cathéters-guides ne sont toutefois pas entièrement satisfaisants, la pratique a en effet montré que si l'on pouvait aisément courber la tête grâce au dispositif de traction, il n'était pas toujours possible de provoquer le redressement de celle-ci, la force élastique de rappel du ressort étant trop faible. Un autre inconvénient de ces cathéters-guides réside dans le fait que malgré son faible diamètre le tube reliant la tête au boîtier de commande n'est pas suffisamment flexible.

Enfin, un autre inconvénient de ces cathéters-guides réside dans le fait que la tête est fixée mécaniquement sur le tube et que l'on ne peut pas garantir de façon absolue que lors du retrait du cathéter-guide une séparation de la tête n'intervient pas nécessitant une opération chirurgicale délicate pour ensuite la récupérer.

La présente invention a pour objet un cathéter-guide du type de celui décrit dans la demande EP-86115666.1 qui n'en présente pas les inconvénients et qui permette en outre de limiter les risques de blessures des vaisseaux sanguins dans lesquels il est introduit et d'obtenir des flexibilités de valeurs différentes suivant les portions envisagées de la partie du cathéter-guide reliant la tête au boîtier de commande.

Le présent cathéter-guide est défini par les caractéristiques des revendications annexées.

Le dessin annexé illustre schématiquement et à titre d'exemple une forme d'exécution du cathéter-guide selon la présente invention.

La figure 1 en est une vue en coupe longitudinale partielle, le boîtier de commande étant en position accouplée.

La figure 2 en est une vue en coupe longitudinale partielle, le boîtier de commande étant enlevé et remplacé par une rallonge.

La figure 3 est une vue à plus grande échelle de la tête du cathéter-guide et d'une partie de son organe tubulaire reliant cette tête au boîtier respectivement à la rallonge.

Les figures 4 et 5 sont des vues à plus grande échelle de variantes d'exécutions du cathéter-guide.

La figure 6 illustre en coupe un détail du découpoir du cathéter-guide.

Les figures 7 et 8 illustrent en coupe une variante du cathéter-guide, l'organe tubulaire étant relié au boîtier de commande respectivement à la rallonge.

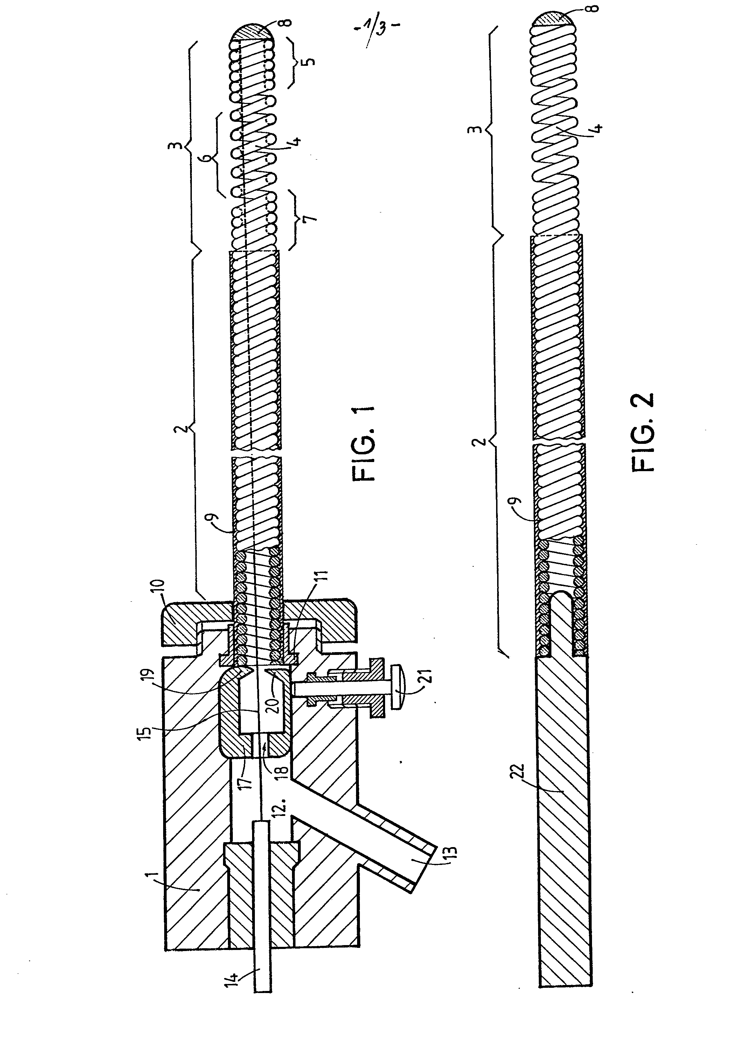

La forme d'exécution du cathéter-guide illustrée aux figures 1 à 3 comporte un boîtier de commande 1 relié de façon amovible à l'extrémité d'un élément tubulaire 2 flexible et de faible diamètre destiné à être introduit en tout ou partie dans le réseau de vaisseaux sanguins d'un patient, dont l'autre extrémité est munie d'une tête orientable 3.

Cette tête orientable ou antenne 3 est réalisée par une portion de ressort spiral 4 présentant trois zones distinctes, une première zone terminale 5 où les spires du ressort 4 sont jointives, une zone médiane 6 où les spires dudit ressort 4 sont disjointes, c'est-à-dire qu'elles ménagent un espace entre elles permettant une liaison directe entre l'extérieur du ressort 4 et son espace tubulaire central, et enfin, une troisième zone 7 où les spires de ce ressort 4 sont à nouveau jointives. La zone terminale 5 et la troisième zone 7 de cette tête 3 définissent ainsi un élément tubulaire fermé, voire semi-étanche au moins.

La zone terminale 5 de cette tête est fermée par une pièce ou bouton 8 soudée sur la dernière spire du ressort 4.

Les dimensions de cette tête 3 sont importantes pour son utilisation, sa longueur est généralement comprise entre 5 et 20 millimètres tandis que son diamètre extérieur est compris entre 0,2 et 0,5 millimètre, de préférence environ 0,4 millimètre. Le diamètre du fil dont est réalisé le ressort est lui de l'ordre de 0,1 millimètre de sorte que l'espace central tubulaire de cette tête est généralement de l'ordre de 0,1 à 0,3 millimètre, de préférence environ 0,2 millimètre.

Cette tête 3 présente ainsi d'une part une zone médiane 6 permettant comme on le verra plus loin le passage d'un fluide de

- 4 -

l'espace tubulaire interne du ressort 4 vers l'extérieur de la tête et donc dans un vaisseau sanguin, et d'autre part une flexibilité importante, notamment due à sa partie médiane 6 à spires disjointes qui lui permet d'être facilement orientable comme on le verra plus loin.

Cette tête est fixée par l'extrémité de sa troisième zone 7 sur un élément tubulaire 2 également constitué par un ressort à spires jointives, de préférence par la continuation du même ressort 4 que celui constituant la tête 3.

La longueur de cet élément tubulaire 2 est de l'ordre de 1 mètre et le diamètre extérieur du ressort qui le constitue est du même ordre de grandeur que celui de la tête, généralement légèrement plus faible, de 0,05 à 0,1 millimètre plus faible. En effet, cet élément tubulaire 2 comporte un revêtement externe 9 en matière plastique d'une épaisseur pouvant varier entre quelques microns et 0,3 millimètre. Ce revêtement externe 9 est obtenu par déposition en phase gazeuse de la matière plastique sur le ressort 4 et permet de rendre cet élément tubulaire complètement étanche et lisse.

De plus, cet élément tubulaire 3 est susceptible de se courber et de se redresser facilement du fait de sa réalisation qui lui confère une grande souplesse et une grande flexibilité.

Ces qualités font qu'il est plus facile aux praticiens de faire pénétrer le cathéter-guide dans un vaisseau sanguin du patient, sa surface étant lisse et sa flexibilité grande, les risques de causer des lésions à ces vaisseaux sanguins sont fortement réduits.

L'extrémité libre de cet élément tubulaire 2 est fixée de façon amovible mais étanche au boîtier de commande 1, dans l'exemple illustré par un accouplement à vis et écrou 10 comportant un joint ou presse-étoupe 11. La cavité intérieure 12 de ce boîtier 1 communique avec l'organe tubulaire central de l'élément 2 et de la tête 3 et peut être alimenté en liquide de contraste par exemple par un canal 13.

Le boîtier de commande comporte encore un organe de manoeuvre 14, coulissant axialement dans ce boîtier de façon étanche, relié par un organe de traction 15 à un point excentré de l'extrémité libre de la tête 3 du cathéter-guide. Cet organe de traction 15 s'étend à l'intérieur du boîtier 1, de l'élément tubulaire 2 et de la tête 3. Il présente une portion très fine et souple 16, d'un diamètre de l'ordre de 1 à 5 centième de millimètre s'étendant sur la longueur de la tête 3 et une portion moins fine et plus rigide 15 reliant cette portion 16 à l'organe de manoeuvre, d'un diamètre de 1 à 2 dixième de millimètres.

Ainsi, l'opérateur en tirant axialement sur l'organe de manoeuvre 14 peut provoquer une courbure de la tête 3, courbure qui n'est pas influencée ou rendue difficile par l'organe de traction 15, 16 car dans cette zone celui-ci est très souple et flexible.

Lorsque l'opérateur veut redresser la tête 3, s'il ne suffit pas de relâcher l'organe de manoeuvre 14 la force de friction entre l'organe de traction 15 et l'élément tubulaire 2 étant trop forte pour que l'élasticité ressort de la tête permette un tel redressement, l'opérateur repousse l'organe de manoeuvre 14 vers le boîtier provoquant un déplacement de la partie de fort diamètre 15 de l'organe de traction dans l'élément tubulaire 2 contre la force de friction. L'élasticité propre de la tête 3 ne doit alors plus que tendre la portion fine et très flexible 16 de cet organe de traction ce qui ne représente pratiquement aucune résistance à vaincre.

Grâce à ces deux caractéristiques nouvelles et originales, élément tubulaire 2 réalisé en ressort spiral recouvert d'une gaine de matière plastique et organe de traction très souple à son extrémité et plus rigide dans le reste de sa longueur, le présent cathéter-guide remédie aux inconvénients des dispositifs similaires existants et permet d'atteindre les buts essentiels que s'était fixés la titulaire.

Comme dans les cathéters-guides existants, le boîtier de commande 1 comporte également un découpoir constitué par une cisaille 17, logée dans le boîtier 1 comportant un perçage 18 donnant passage à l'organe de traction 15 et deux mâchoires l'une fixe 19 et l'autre 20 déplaçable contre son élasticité propre à l'aide d'un poussoir 21 accessible depuis l'extérieur du boîtier.

Grâce à ce découpoir l'opérateur peut, lorsque le cathéter-guide est introduit en position de service dans le vaisseau sanguin du patient, couper l'organe de traction ce qui permet ensuite de déconnecter le boîtier 1 de l'élément tubulaire 2 et de fixer par emboîtage à l'extrémité de ce dernier un manche plein ou rallonge 22 comme illustré à la figure 2. Ce manche 22 présente un diamètre extérieur sensiblement égal à celui de l'élément tubulaire 2.

En variante, la partie avant de l'élément tubulaire 2 ` peut être munie de façon connue d'un ballonnet extensible pouvant être alimenté par du liquide qui lui est acheminé par un conduit annulaire entourant la gaine 9 et relié dans le boîtier de commande 1 à un conduit d'alimentation particulier.

Suivant les applications envisagées, il est souvent souhaitable que l'élément tubulaire 3 puisse présenter des degrés de flexibilité différents, plus rigide à proximité du boîtier de commande 1 et plus flexible à proximité de la tête 3.

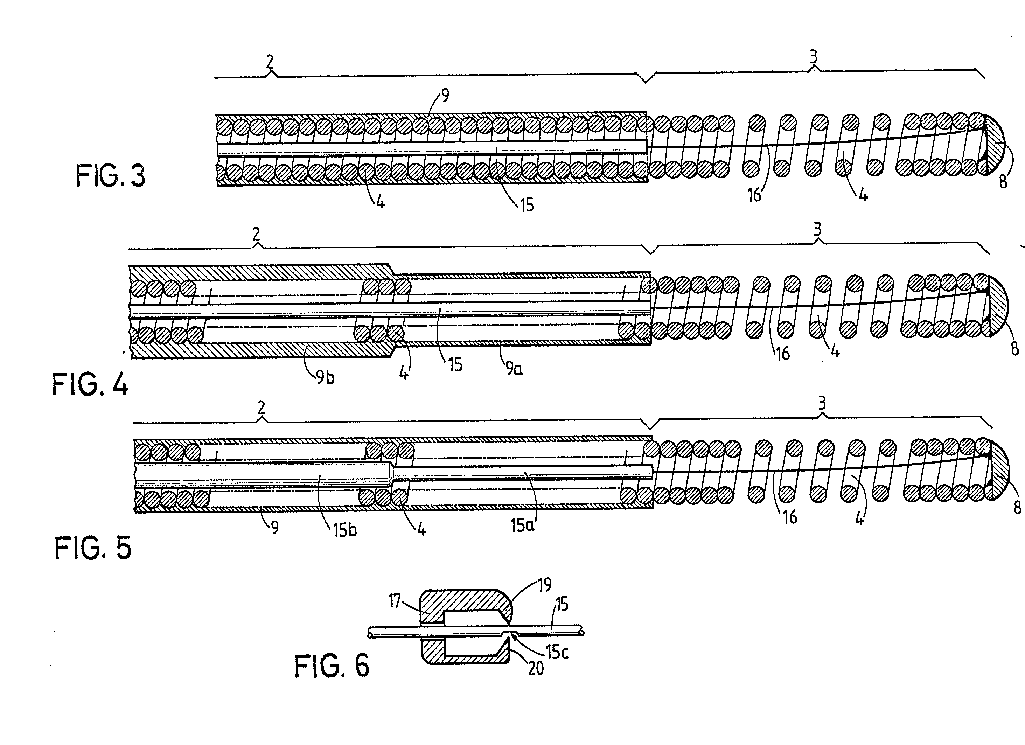

Ceci peut être obtenu comme dans la variante illustrée à la figure 4 par un revêtement ou gaine d'épaisseur variable. La partie frontale de l'élément tubulaire 2 reçoit une gaine mince 9a tandis que la partie arrière de cet élément 2 est munie d'une gaine 9b plus épaisse augmentant la rigidité de cet élément 2.

Suivant les caractéristiques de flexibilité désirée de l'élément tubulaire 2 plusieurs différences d'épaisseur (3 à 5) de ce revêtement 9 peuvent être envisagées. Il est également possible par ce moyen de donner à cet élément tubulaire une plus grande rigidité dans ses parties médianes que dans sa partie avant ou arrière.

Dans une autre variante illustrée à la figure 5 on obtient ces différences de flexibilité de l'élément tubulaire 2 par des différents diamètres de l'organe de traction, la partie 15 de celui-ci s'étendant à l'intérieur de l'élément tubulaire 2 pouvant présenter des parties 15a, 15b de diamètres différents. Plus la section de l'organe de traction est importante, plus la rigidité de l'élément tubulaire est grande.

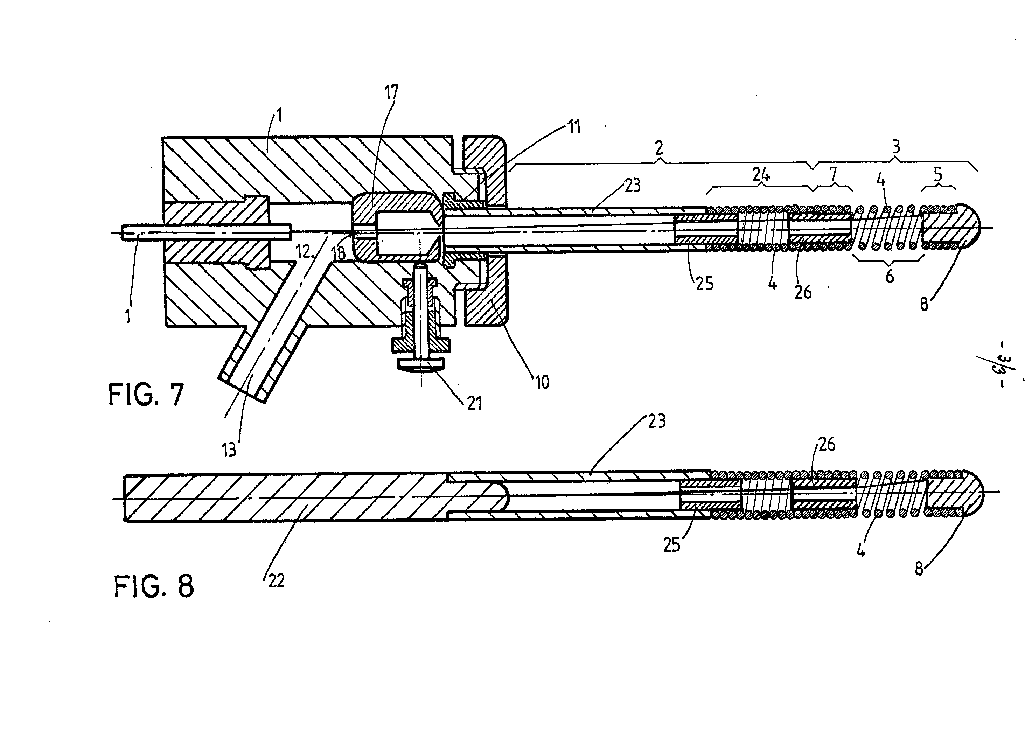

Dans certains cas où le diamètre de l'organe de traction au voisinage du découpoir serait trop important pour permettre un sectionnement aisé de celui-ci, on peut, comme illustré à la figure 6, prévoir une diminution locale 15c de la section de cet organe de traction 15 ou un diamètre plus faible à cet endroit.

Les principaux avantages obtenus par le cathéter-guide objet de la présente invention, sont :

1. une souplesse et une flexibilité accrue de l'ensemble de l'instrument.

2. de faciliter, ou même de rendre possible le redressement de la tête de l'instrument après que celui-ci ait été coudé.

3. d'éviter des blessures des parois des vaisseaux sanguins.

4. de pouvoir pénétrer des réseaux artériels de petits calibres et plus tourmentés.

La seconde forme d'exécution illustrée aux figures 7 et 8 comporte un organe tubulaire 2 reliant la tête 3 au boîtier de commande 1 respectivement à la rallonge 22 qui comporte une première partie 23, reliée par une extrémité au boîtier de commande 1, formée par un tube fin et flexible; et une seconde partie 24, reliée à la tête 3, constituée par un ressort à boudin à spires jointives.

Cette seconde partie 24 est composée d'un ressort à boudin cylindrique à spires jointives recouvert d'une enveloppe plastique dont la base est enmanchée sur un embout percé 25 lui-même chassé à l'intérieur du tube 23 et dont l'autre extrémité est enmanchée sur un embout percé 26, ce dernier étant chassé à l'intérieur de la tête 3.

0247371

On obtient grâce à cette réalisation une plus grande souplesse de l'extrémité de l'organe tubulaire.

0247371

- 1 -

<u>REVENDICATIONS</u>

1.      Cathéter-guide comprenant; une tête formée par un ressort à boudin présentant une zone médiane où les spires de ce ressort sont disjointes située entre deux zones où les spires de ce
ressort sont jointives, l'extrémité libre de cette tête étant obturée par un organe d'obturation; un élément tubulaire reliant cette tête à un boîtier de commande auquel il est fixé de façon amovible; ainsi qu'un dispositif de commande de la flexion de la tête
comportant un organe de manoeuvre coulissant dans le boîtier relié
par un organe de traction traversant ledit boîtier, l'élément tubulaire et la tête à un point excentré de l'extrémité libre de
cette tête; et un dispositif de sectionnement de l'organe de traction logé dans ledit boîtier de commande; caractérisé par le fait
que l'élément tubulaire est constitué sur une partie au moins de
sa longueur par un ressort à boudin à spires jointives recouvert
d'un revêtement étanche en matière plastique.

2.      Cathéter-guide selon la revendication 1, caractérisé
par le fait que le ressort à boudin formant la tête et celui de
l'élément tubulaire sont un seul et même ressort.

3.      Cathéter-guide selon l'une des revendications précédentes, caractérisé par le fait que la partie avant de l'organe de
traction s'étendant sur au moins une partie de la longueur de la
tête présente une section plus faible que le reste de cet organe
de traction.

4.      Cathéter-guide selon la revendication 3, caractérisé par
le fait que le diamètre de la partie avant de l'organe de traction,
formé par un fil métallique est compris entre 1 et 5 centième de
millimètre, permettant la transmission d'un effort de traction
mais non d'un effort de poussée et n'influençant pas les caractéristiques de flexibilité propres de la tête.

5.     Cathéter-guide selon l'une des revendications précédentes, caractérisé par le fait que la partie de l'organe de traction s'étendant à l'intérieur de l'élément tubulaire comporte plusieurs portions de diamètres différents les unes des autres.

6.     Cathéter-guide selon la revendication 5, caractérisé par le fait que les diamètres des différentes parties de l'organe de traction vont en diminuant du boîtier en direction de la tête.

7.     Cathéter-guide selon la revendication 5 ou la revendication 6, caractérisé par le fait que l'organe de traction présente un rétrécissement au droit des mâchoires du découpoir.

8.     Cathéter-guide selon l'une des revendications précédentes, caractérisé par le fait que le revêtement en matière plastique de l'élément tubulaire présente une épaisseur constante.

9.     Cathéter-guide selon l'une des revendications 1 à 7, caractérisé par le fait que le revêtement en matière plastique de l'élément tubulaire présente plusieurs épaisseurs différentes.

10.     Cathéter-guide selon la revendication 9, caractérisé par le fait que l'épaisseur du revêtement en matière plastique de l'élément tubulaire est dégressif depuis le boîtier en direction de la tête.

11.     Cathéter-guide selon l'une des revendications précédentes, caractérisé par le fait que la totalité de l'élément tubulaire est constituée par un ressort à boudin à spires jointives.

12.     Cathéter-guide selon l'une des revendications 1 à 10, caractérisé par le fait que seule la partie avant de l'élément tubulaire est formée par un ressort à boudin à spires jointives.

FIG. 1

FIG. 2

0247371

-2/3-

FIG. 3

FIG. 4

FIG. 5

FIG. 6

FIG. 7

FIG. 8

Office européen
des brevets

**RAPPORT DE RECHERCHE EUROPEENNE**

0247371
Numero de la demande

EP 87 10 6172

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | US-A-4 215 703 (J. WILSON) * Figure 4; colonne 3, lignes 26-41; colonne 2, lignes 50-64 * | 1,2 | A 61 M 25/00 A 61 B 1/00 |
| A | US-A-4 543 090 (W. McCOY) * Figures 1-3 * | 1 | |
| A | US-A-4 456 017 (M. MILES) * Colonne 4, lignes 1-19,37-42; figures 1,2 * | 1-12 | |
| A | GB-A-2 130 885 (ELVEN PRECISION LTD) * Figures 2,3; page 1, lignes 73-94 * | 1 | |
| A | US-A-3 528 406 (JECKEL et al.) * Figures 1,2 * | 1,3-6 | DOMAINES TECHNIQUES RECHERCHES (Int. Cl.4) A 61 M |
| A | US-A-4 534 363 (GOLD) * Figures 1,2 * | 1-3 | |

Le présent rapport de recherche a été établi pour toutes les revendications

| Lieu de la recherche | Date d'achèvement de la recherche | Examinateur |
|---|---|---|
| LA HAYE | 29-07-1987 | RAKOWICZ, J.M. |